# EUROPEAN PATENT SPECIFICATION

(11) **EP 1 892 236 B1**
(45) Date of publication and mention of the grant of the patent: **22.06.2011**
(21) Application number: 07113600.6
(22) Date of filing: 01.08.2007
(51) Int. Cl.: C07C 219/06, A61K 8/41, A61Q 19/10, A01N 37/00

(54) **Iodide salts of dimethylaminoethanol fatty acid esters with bacteriostatic, mycostatic, yeaststatic and/or microbicide activity for use in cleansing or purifying formulations**
Iodsalze von Dimethylaminoethanol Fettsäureester mit bakteriostatischer, myostatischer, hefestatischer und/oder mikrobizider Aktivität zur Verwendung in Wasch- oder Reinigungsformulierungen
Iodures d'éster d'acides gras de diméthylaminoéthanol dotés d'une activité bactériostatique, mycostatique, levurostatique et/ou microbicide pour une utilisation dans des formulations de nettoyage ou de purification

(30) Priority: 03.08.2006 IT MI20061551
(43) Date of publication of application: 27.02.2008
(73) Proprietor: Scharper S.p.A., 20121 Milano (IT)
(72) Inventor: Razzano, Gianni, 21030 Fino Mornasco (IT)
(74) Representative: Gervasi, Gemma

(56) References cited:
- JP-A- 2005 015 377
- US-B1- 6 420 330
- BIEN M ET AL: "Antifungal Activity of Some Model soft Lysosomotropic Compounds" BULLETIN OF THE POLISH ACADEMY OF SCIENCES. BIOLOGICAL SCIENCES, PANSTWOWE WYDAWNICTWO NAUKOWE, WARSAW,, PL, vol. 43, no. 2, 1995, pages 105-112, XP009091649 ISSN: 0867-1656
- PROTIVA MOROSLAV ET AL: "Potential cerebral stimulants: esters of 2-(dimethylamino)ethanol with some lipophilic carboxylic acids" COLLECTION OF CZECHOSLOVAK CHEMICAL COMMUNICATIONS, INSTITUTE OF ORGANIC CHEMISTRY & BIOCHEMISTRY, PRAGUE, CZ, vol. 55, no. 5, 1990, pages 1278-1289, XP002969314 ISSN: 0010-0765
- B. AHLSTRÖM ET AL: "Long-chain alkanoylcholines, a new category of soft antimicrobial agents that are enzymatically degradable" ANTIMICROBIAL AGENTS AND CHEMOTHERAPY, vol. 39, no. 1, 1995, pages 50-55, XP002458038

## Description

### FIELD OF THE INVENTION

The present invention relates to agents with bacteriostatic, mycostatic and yeaststatic and/or microbicide activity to be used in purifying or cleansing formulations for the skin, mucosa and ear, and to a process for preparing said agents.

### STATE OF THE ART

US 6,420,330 describes microbicides consisting of quaternary carboxylic acid alkanolamine ester salts and more particularly:
- alkyl triethanolamine mono-, di-, and triester salts,
- dialkyl diethanolamine mono- and diester salts,
- trialkyl ethanolamine monoester salts.

These products are obtained using essentially a 3-step process:
1) Mono-, di- or triesterification of the corresponding mono-, di- or trialkanolamine by transesterification,
2) subsequent quaternization by means of an alkyl halide, thus obtaining the corresponding quaternary salt of the mono-, di- or triester of mono-, di- or trialkanolammmonium halide,
3) optional conversion into the desired salt by exchange reaction with an alkaline or alkaline earth salt having the desired anion (i.e. formate, acetate, tartrate, dicarboxylate, a halide different from that present at the end of step (2), sulfate or nitrate).

This synthesis presents a series of drawbacks, primarily the need to form a quaternary ammonium salt by reaction with alkylating agents such as alkyl halides which are toxic and the need to operate, at least in the first two steps, in the presence of organic solvents.

Within the fields of cosmetic formulations and skin cleansing, there is an increasing need for the provision of substances that are as well-tolerated and biocompatible as possible and also that can be synthesized while avoiding or at least reducing organic solvent and toxic reagent use, hence using reagents that are themselves biocompatible.

A reagent that can be thus classed is, for example, dimethylaminoethanol, i.e. a substance which is found at low concentrations in the human brain and in certain foods (sardines and anchovies) and which is an ingredient recently used in cosmetic formulations as a skin toner with anti-wrinkle activity.

### SUMMARY

The Applicant has now prepared new salts of dimethylaminoethanol fatty acid esters of general formula (I) 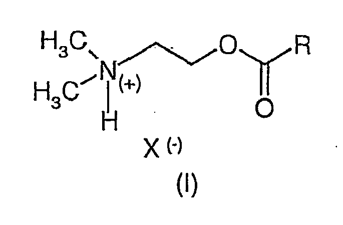 in which R is a C₆-C₁₆ linear or branched alkyl radical, X⁻ = l⁻, and which, as well as being biocompatible, has bacteriostatic, mycostatic, yeaststatic and/or microbicide activity.

In addition to have said properties, these salts are sufficiently water soluble with surfactant properties so that said aqueous solutions, which contain them, form a substantial amount of foam.

These salts can hence be advantageously used in purifying or cleansing formulations of the skin, mucosa and the auricle.

Therefore a further aspect of the present invention are the said cleansing or sanitizing formulations for the skin, mucosa or ear comprising at least one of said agents with bacteriostatic, mycostatic, yeaststatic and/or microbicide activity.

A further aspect of the present invention is a process for preparing the salt of formula (I) which comprises the following steps:
a) esterifying dimethylaminoethanol with a halide of formula RCOX', wherein X' is a halogen selected from chloride or bromide, optionally in the presence of a hydrogen ion acceptor, thus obtaining the ester of formula (II), 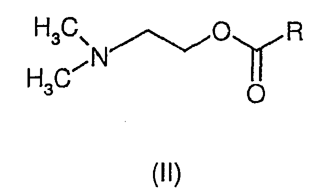 wherein R has the aforesaid meanings,
b) salifying the ester of formula (II) by treating the reaction mixture derived from step (a) in order to obtain the salts of formula (I).

### DESCRIPTION OF THE FIGURE

Figure 1 represents the IR spectrum of myristate of ethyl dimethylammonium iodide prepared as described in example 3.
Figure 2 shows the killing curve with respect to *P.aeruginosa* obtained for Control, 1,2,4xMIC of myristate of ethyldimethylammonium iodide.
Figure 3 shows the killing curve with respect to *Staphylococcus aureus* OXA-S obtained for Control, 1,2,4xMIC of myristate of ethyldimethylammonium iodide.
Figure 4 shows the killing curve with respect to *Staphylococcus aureus* OXA-R obtained for Control, 1,2,4xMIC of myristate of ethyldimethylammonium iodide.
Figure 5 shows the killing curve with respect to *Staphylococcus epidermidis* OXA-R obtained for Control, 1,2,4xMIC of myristate of ethyldimethylammonium iodide.
Figure 6 shows the killing curve with respect to *Staphylococcus epidermidis* OXA-S obtained for Control, 1,2,4xMIC of myristate of ethyldimethylammonium iodide.
Figure 7 shows the killing curve with respect to *Streptococcus pyogens* obtained for Control, 1,2,4xMIC of myristate of ethyldimethylammonium iodide.
Figure 8 shows the killing curve with respect to *Candida albicans* obtained for Control, 1,2,4xMIC of myristate of ethyldimethylammonium iodide

### DETAILED DESCRIPTION OF THE INVENTION

Briefly, the invention concerns salts of formula (I). Salts wherein R is a C₁₀-C₁₄ linear or branched alkyl radical are preferred. More preferably, R is a C₁₃ linear alkyl radical, i.e. the fatty acid of the ester is myristic acid.

According to the invention, X⁻ is iodide.

In this respect the salts of the invention presenting an iodide anion can be prepared by the process of the invention by carrying out the steps (a) and (b) in an aqueous solvent and using sodium hydroxide in step (a) as hydrogen ion acceptor. In this case, after adding hydriodic acid, the iodide salt precipitates on cooling and can be separated from the reaction mixture by filtration.

The skin purifying or cleansing formulations of the present invention preferably contain the salts of the present invention at concentrations between 0.001 and 1%, more preferably at concentrations between 0.05 and 0.1% by weight on the total weight of the formulation.

The cleansing or purifying formulations for the skin and mucosa of the present invention are preferably water based and preferably contain additives normally used in cleansing formulations such as cationic, amphoteric and non-ionic surfactants, and preservatives etc.

The cleansing or purifying formulations suitable for skin of the present invention are preferably in the form of anti-blackhead and anti-acne soaps, lotions, emulsions, or in the form of anti-dandruff shampoos.

The cleansing or purifying formulations suitable for the mucosa are suitable in particular as cleansers for intimate hygiene and are in the form of lotions or fluid emulsions.

The ear cleansing or purifying formulations in accordance with the present invention are preferably in the form of aqueous solutions, more preferably physiological solutions, and can also contain auxiliary surfactants.

The following examples of the preparation of the salts of formula (I) and the relative evaluations of bacteriostatic, mycostatic, yeaststatic and/or microbicide activity are provided by way of non-limiting illustration.

### EXAMPLE 1 - Preparation of laurate of ethyl dimethylammonium iodide

A solution of 8.9 g of dimethylaminoethanol and 4.0 g of sodium hydroxide in 70 g of water are prepared. 21.8 g of lauroyl chloride are added slowly at ambient temperature to the aforesaid solution maintained under stirring. When addition is complete, the reaction mixture is heated at 80°C for 2 hours until it becomes clear. It is then cooled to ambient temperature and 22.4 g of 57% w/w hydriodic acid are added. It is then cooled to ambient temperature and a crystalline precipitate is obtained which is filtered off and dried: reaction yield: 85%.

### EXAMPLE 2 - Preparation of myristate of ethyl dimethylammonium iodide

Following the same operating method as used in the preceding example with the only difference that the lauroyl chloride is replaced by the same amount of myristyl chloride, myristate of ethyl dimethylammonium iodide is obtained. The final product, separated from the reaction mixture by filtration, is recrystallized from an ethanol:water mixture (1:1) and dried at ambient temperature under vacuum: melting point of the product: 54°C; reaction yield: 75%.

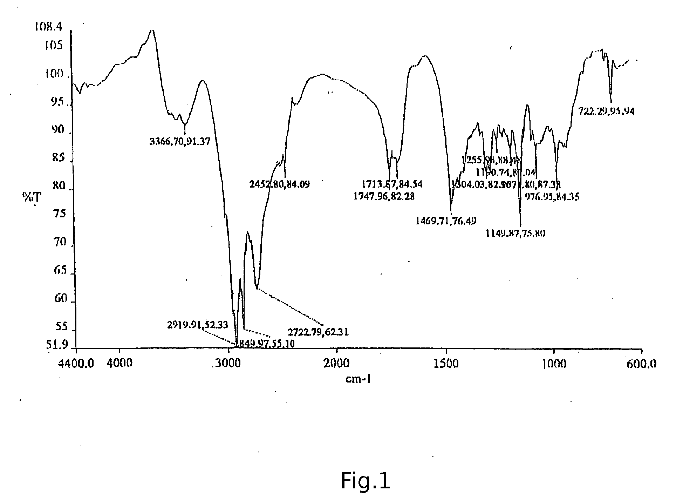

The crystallized product is analysed with a Perkin Elmer IR spectrometer, the relative spectrum being represented in Figure 1 from which it can be seen that:
□ The peaks at around 3000 cm⁻¹ relate to the aliphatic chain of the ester
□ The peaks at 3366 and 2452 cm⁻¹ relate to the quaternary nitrogen, and
□ The peak at 1713 cm⁻¹ relates to the CO peak present in the ester group.

### EXAMPLE 3 - Preparation of laurate of ethyl dimethylammonium chloride (comparative)

8.9 g of dimethylaminoethanol are dissolved in 40 g of toluene. To this solution 5 g of Amberlite IRA 400 cationic resin (cationic resin by cross-linking of benzyl-dimethyl(2-hydroxyethyl) ammonium) are added under stirring. 21.8 g of lauroylchloride are slowly added. The mixture is then heated under reflux for 2 hours. After cooling, the mass solidifies. 25 g of ethanol are then added, and after heating to 70°C the resin is filtered off and the mixture cooled to ambient temperature. A crystalline precipitate is then obtained which is separated from the reaction mixture and dried under vacuum at ambient temperature in an oven.
Reaction yield: 75%.

### EXAMPLE 4 - Preparation of laurate of ethyl dimethylammonium bromide (comparative)

Following the same operating method as used in example 3, with the only difference that lauroyl chloride is replaced by the same amount of lauroyl bromide, the compound of the title was obtained. Reaction yield: 70%.

### EVALUATION OF BACTERIOSTATIC, MYCOSTATIC AND YEASTSTATIC ACTIVITY

Tests were conducted by using standard turbidimetric protocols of MIC (Minimal Inhibitory Concentration) evaluation of the myristate of ethyl dimethylammonium iodide on both collection colonies and on other available strains. The values were expressed in ppm and represent the concentration limits in aqueous solution of said salt, above which no more biological growth is apparent.

| Strain | MIC (ppm) |
|---|---|
| *Candida Albicans* ATCC10231 | 10 |
| *Bacillus Subtilis* ATTCC 6633 | 2 |
| *Staphylococcus Aureus* ATCC 25923 | 3 |
| *Pseudomonas Aeruginosa* | 25 |
| *Escherichia Coli* ATTC11229 | 15 |
| *Aspergillus Fumigatus* | 15 |

The above values, when expressed in % concentration (w/w), are between 0.002 and 0.025%.

### EVALUATION OF ANTIMICROBIAL ACTIVITY OF MYRISTATE OF ETHYLDIMETHYLAMMONIUM IODIDE

A study was carried out with the aim of characterizing the antimicrobial activity of myristate of ethyl dimethylammonium iodide against microorganisms belonging to the following species:
- oxacillin/methicillin-resistant and -sensitive *Staphylococcus aureus,* briefly indicated as Oxa-R *S.aureus* and Oxa-S *S.aureus* respectively;
- oxacillin/methicillin-resistant and -sensitive *Staphylococcus epidermidis,* briefly indicated as Oxa-R *S. epidermidis* and Oxa-S *S. epidermidis* respectively;

### - Pseudomonas aeruginosa

### - Candida albicans

### - Streptococcus pyogenes.

The control strain for all staphylococcus microorganisms was *Staphylococcus aureus* ATCC 25923, while for *Pseudomonas aeruginosa* the control strain was *Pseudomonas aeruginosa* ATCC 27853.

### Evaluation of MIC and MMC

The values of Minimal Inhibitory Concentrations (MIC) and Minimal Microbicide Concentrations (MMC) were obtained through either the method of liquid broth microdilution or the method of agar dilution according to the Guidelines of National Committee for Clinical and Laboratory Standards (NCCLS).

Such methods provide for inoculum of 1-5 x 10⁵ CFU/ml in wells of a microplate or in agar containing graduated concentrations of myristate of ethyldimethyl ammonium iodide, dissolved in the growth soil suitable for microorganism test.

Specifically, in order to determine the values of MIC the following broths were used:
- Mueller Hinton added with cations for *S.aureus, S.epidermidis* and *P. aeruginosa,*
- Mueller Hinton supplemented with mutton blood (5%) for *Streptococcus pyogenes,*
- Sabouraud for *Candida albicans,*

After 18 hours of incubation, the lowest concentration of myristate capable of inhibiting the growth of the microorganism corresponded to MIC. The obtained values are shown in the Table below.

MMC, defined as the lowest concentration of myristate of ethyldimethyl ammonium iodide capable of determining the death of 99,9% of the initial microbial population, was determined through microdilution method, by seeding an aliquot from each well of the microplate used for the evaluation of MIC on plates of suitable soil and subsequently by incubating for 18-24 hours in case of bacteria (*S.aureus, S.epidermidis, P. aeruginosa* and *Streptococcus pyogenes)* and 48-72 hours in case of fungus *Candida albicans.*

Specifically, in order to determine the values of MMC the following soils were used:
- Mueller Hinton for *S.aureus, S.epidermidis* and *P. aeruginosa,*
- Mueller Hinton supplemented with mutton blood (5%) for *Streptococcus pyogenes,*
- Sabouraud for *Candida albicans,*

The obtained values are shown in the Table below.

**Table: MIC and MMC of myristate of ethyl dimethylammonium iodide**

| Microorganism | MIC (µg/ml) | MMC (µg/ml) |
|---|---|---|
| Control strain: *S.aureus* ATCC 25923 | 128 | 128 |
| Oxa-R *S.aureus* | 64 | 128 |
| Oxa-R *S. epidermidis* | 64 | 256 |
| Oxa-S *S. epidermidis* | 32 | 128 |
| Oxa-S *S. epidermidis* | 64 | 128 |
| | | |
| Control strain: *P. aeruginosa* ATCC 27853 | 32 | >256 |
| *P.aeruginosa* | 32 | >256 |
| | | |
| *Candida* albicans | 16 | 32 |
| *S. pyogenes.* | 32 | 128 |

As it is evident from the Table the salt of the invention has a good inhibiting activity against bacteria and a very good one against fungus *C.albicans.*

As far as MMC is concerned, the salt of the invention shows also a good microbicide activity, particularly against *C.albicans.*

### Killing curves

The killing kinetics were obtained on microbial strains which were representative for each above indicated microbial species. The used concentrations of myristate of ethyl dimethyl ammonium iodide were: 1, 2, 4 x MIC. For each microorganism, the growth curve without addition of myristate (control curve) was obtained. Killing curves in the suitable growth broth containing myristate in the above indicated concentrations were obtained by inoculating a microbial amount of 1-5x10⁵ cfu/ml. After 0, 3, 6, and 24 hours of incubation at 37°C the still present bacteria were counted, by seeding, in duplicate, an aliquot of growth broth in plates containing the suitable soil. For *Candida albicans,* incubation was protracted to 48-72 hours. Antimicrobial activity was deemed as being microbicide, when a decrease of at least 3 log of microbial counts, with respect to initial inoculum, was present, while in case of a decrease lower than 3 log the antimicrobial activity was deemed as being static.

Specifically, in order to obtain the killing curves the following soils were used:
- Mueller Hinton for *S.aureus, S.epidermidis* and *P. aeruginosa,*
- Mueller Hinton supplemented with horse lisate blood (5%) for *Streptococcus pyogenes,*
- Sabouraud for *Candida albicans.*

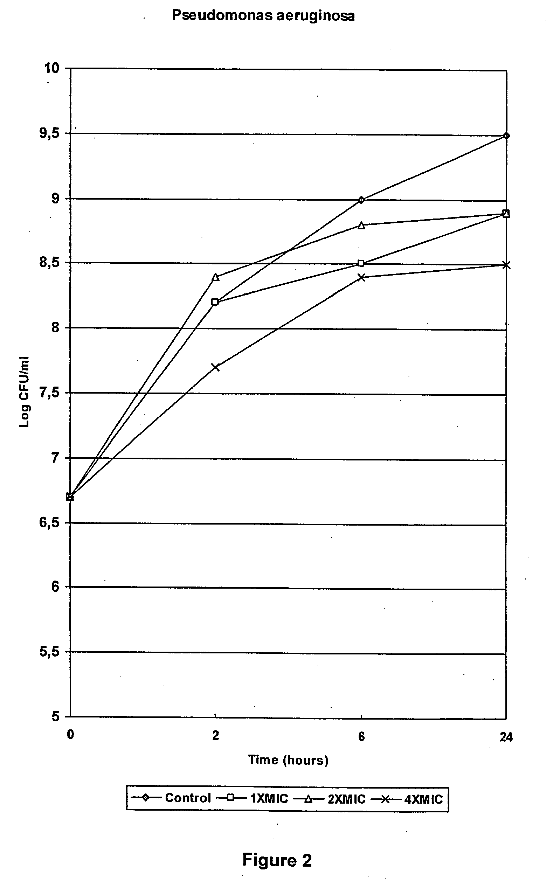

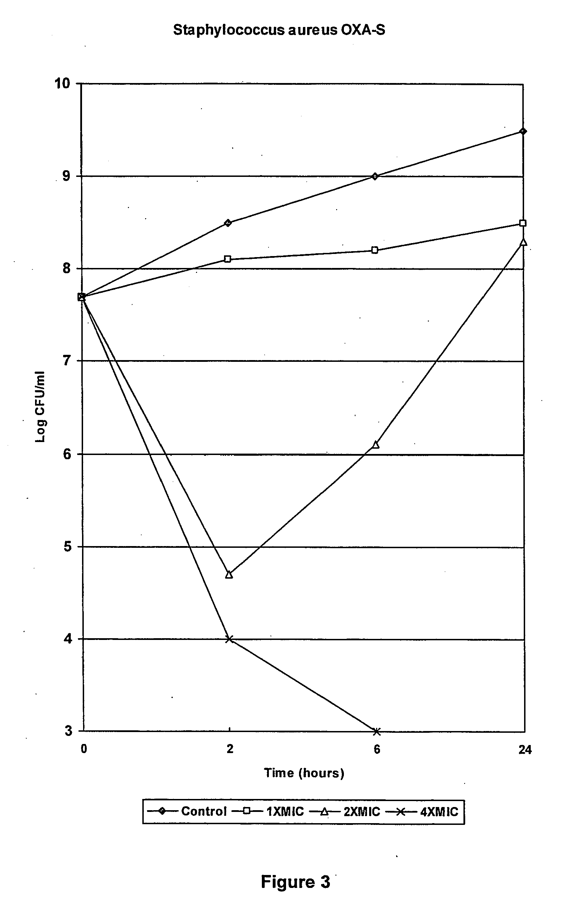

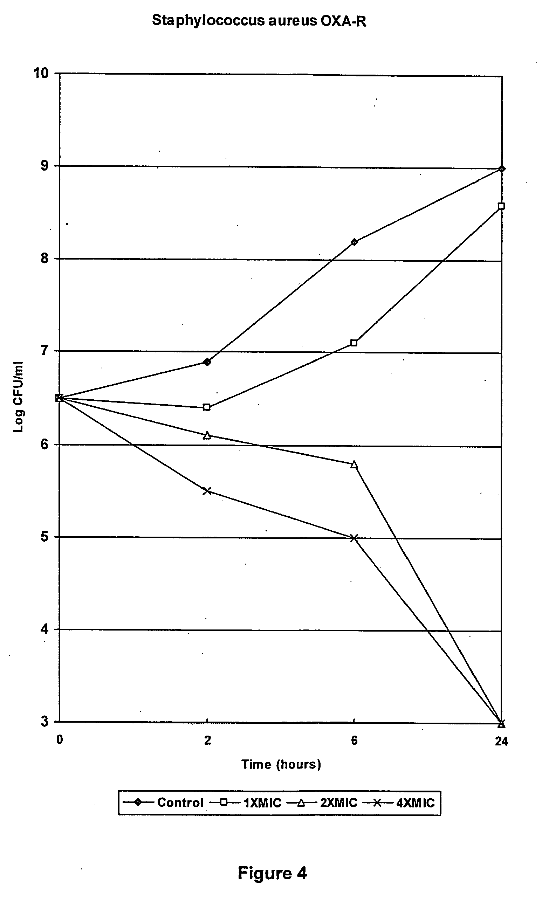

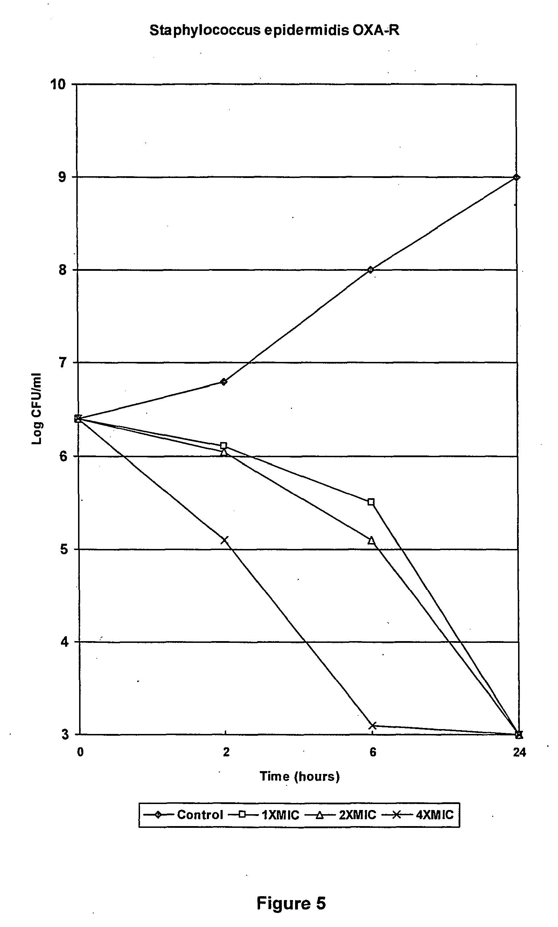

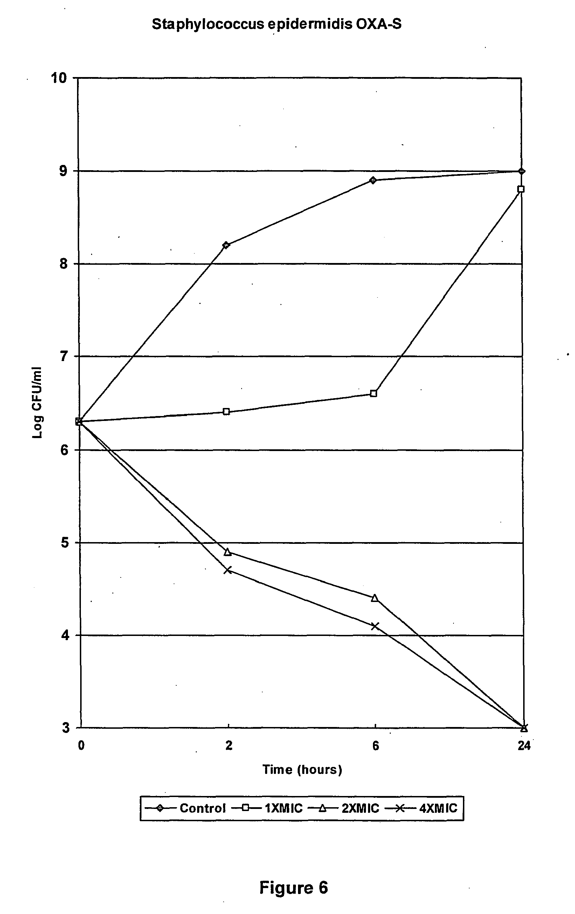

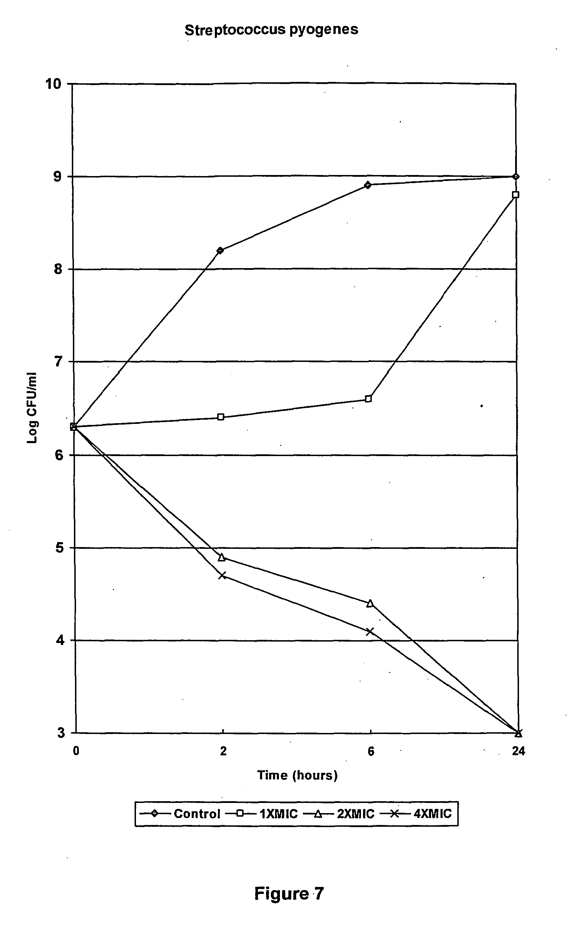

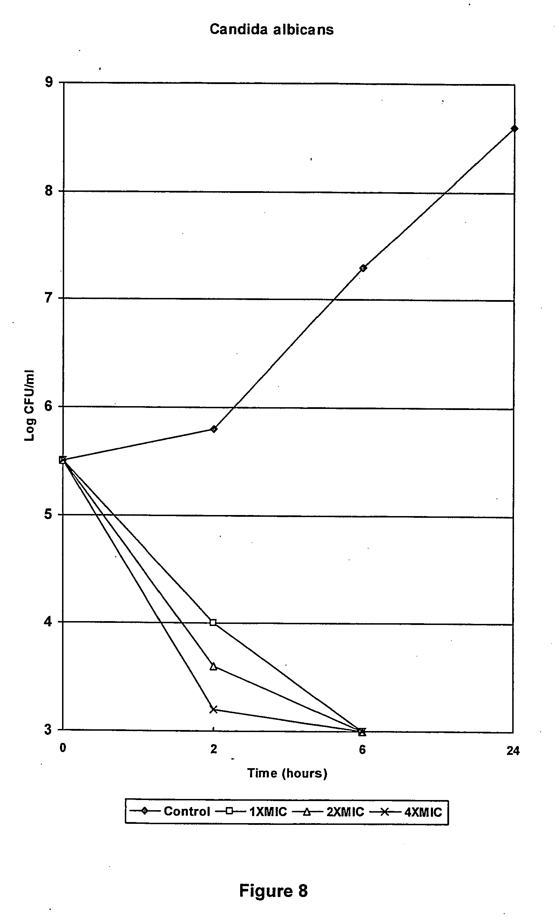

The killing curves are represented in the annexed figures 2-8. As it can be seen from figures 3-7 the salt of the invention shows microbicide activity against tested species of *Staphylococcus* and *Streptococcus* at concentrations of 2xMIC and 4XMIC, and bacteriostatic activity at lower concentration. As far as the activity against *P. aeruginosa* of Figure 2 is concerned, the compound of the invention shows predominantly static antimicrobic activity. Advantageously, the microbicide activity of the salt of the invention against *C. albicans* of figure 8 was high, even at concentration of 1xMIC.

## Claims

1. Salts of dimethylaminoethanol fatty acid esters of general formula (I) 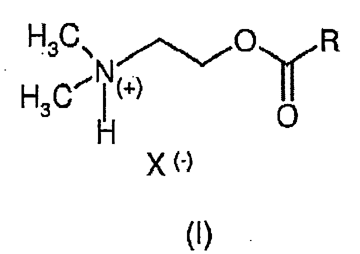 wherein R is a C₆-C₁₆ linear or branched alkyl radical, X⁻ = I⁻.

2. Salts according to claim 1, wherein R is C₁₀-C₁₄ linear or branched alkyl radical.

3. Salts according to claim 2, wherein R is a C₁₃ linear alkyl radical.

4. Use of salts according to any one of claims 1-3 as bacteriostatic, mycostatic, yeaststatic and/or microbicide agents.

5. Formulations comprising at least one salt according to anyone of claims 1-3, wherein said at least one salt is at concentrations between 0.001 and 1% by weight on the total formulation weight.

6. Formulations according to claim 5 wherein said concentration is between 0.05 and 0.1%.

7. Formulations according to claim 5 or claim 6 for cleansing or purifying skin or mucosa, said formulations being water based.

8. Formulations according to anyone of claims 5-7, said formulations being in the form of anti-acne soaps, lotions, or emulsions.

9. Formulations according to anyone of claims 5-7, said formulations being in the form of an anti-dandruff shampoo.

10. Process for preparing salts of general formula (I) comprising the following steps:
a) esterifying dimethylaminoethanol with a halide of formula RCOX', wherein X' is a halogen selected from chloride or bromide, thus obtaining the ester of formula (II), 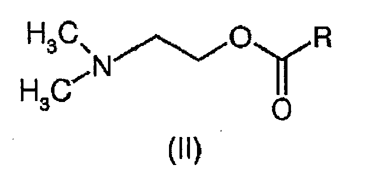 in which R is a C₆-C₁₆ linear or branched alkyl radical,
b) salifying the ester of formula (II) by treating the reaction mixture derived from step (a) with HX" acid, wherein X" = I, in order to obtain the salts of formula (I).

11. Process according to claim 10, wherein step (a) is carried out in the presence of a hydrogen ion acceptor.

12. Process according to claim 11 for preparing the salt of formula (I) in which X⁻ = I⁻, wherein steps (a) and (b) are carried out in an aqueous solvent and in step (a) sodium hydroxide as hydrogen ion acceptor is used, and on completion of step (b) after adding a stoichiometric amount of hydriodic acid, the salt precipitates directly from the aqueous reaction mixture on cooling.

## Patentansprüche

1. Salze von Dimethylaminoethanolfettsäureestern gemäß der allgemeinen Formel (I) 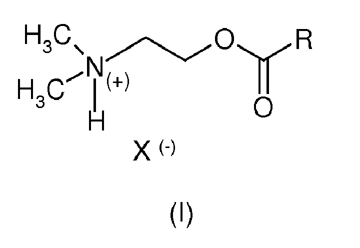 worin R ein lineares oder verzweigtes C₆-C₁₆-Alkylradikal ist und X- = I⁻ ist.

2. Salze nach Anspruch 1, wobei R ein lineares oder verzweigtes C₁₀-C₁₄-Alkylradikal ist.

3. Salze nach Anspruch 2, wobei R ein lineares C₁₃-Alkylradikal ist.

4. Verwendung von Salzen nach einem der Ansprüche 1 bis 3 als bakteriostatische, mykostatische, hefestatische und/oder mikrobizide Mittel.

5. Formulierungen enthaltend wenigstens ein Salz nach einem der Ansprüche 1 bis 3, wobei das wenigstens eine Salz in Konzentrationen zwischen 0,001 und 1 Gew.-% bezogen auf das Gesamtformulierungsgewicht vorliegt.

6. Formulierungen nach Anspruch 5, wonach die Konzentration zwischen 0,05 und 0,1 % beträgt.

7. Formulierungen nach Anspruch 5 oder Anspruch 6 zum Reinigen oder Säubern von Haut oder Schleimhaut, wobei die Formulierungen auf Wasserbasis sind.

8. Formulierungen nach einem der Ansprüche 5 bis 7, wobei die Formulierungen in der Form von Anti-Akne-Seifen, -Lotionen oder -Emulsionen vorliegen.

9. Formulierungen nach einem der Ansprüche 5 bis 7, wobei die Formulierungen in der Form eines Antischuppenshampoos vorliegen.

10. Verfahren zum Herstellen von Salzen der allgemeinen Formel (I), welches die nachfolgenden Schritte umfasst:
a) Verestern von Dimethylaminoethanol mit einem Halogenid gemäß der Formel RCOX', worin X' ein Halogen ausgewählt aus Chlorid oder Bromid ist, so dass ein Ester gemäß der Formel (II) 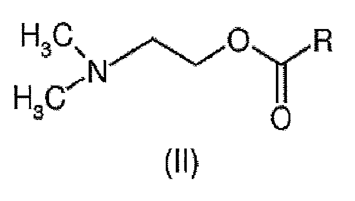 erhalten wird, worin R ein lineares oder verzweigtes C₆-C₁₆-Alkylradikal ist,
b) Überführen des Esters gemäß der Formel (II) in ein Salz durch Behandeln der Reaktionsmischung, welche aus dem Schritt (a) stammt, mit HX"-Säure, worin X" = I ist, um Salze gemäß der Formel (I) zu erhalten.

11. Verfahren nach Anspruch 10, wobei der Schritt (a) in der Gegenwart eines Wasserstoffionenakzeptors durchgeführt wird.

12. Verfahren nach Anspruch 11 zum Herstellen eines Salzes gemäß der Formel (I), wobei X⁻ = I⁻ ist, wobei die Schritte (a) und (b) in einem wässrigen Lösungsmittel durchgeführt werden und in dem Schritt (a) Natriumhydroxid als Wasserstoffionenakzeptor eingesetzt wird, und wobei bei der Beendigung des Schritts (b) das Salz nach der Zugabe einer stöchiometrischen Menge von Iodwasserstoffsäure beim Abkühlen direkt aus der wässrigen Reaktionsmischung präzipitiert.

## Revendications

1. Sels d'esters d'acide diméthylaminoéthanol gras de formule générale (I) 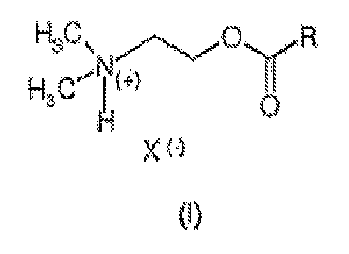 dans laquelle R est un radical alkyle linéaire ou ramifié en C₆ à C₁₆, X⁻ = I⁻.

2. Sels selon la revendication 1, dans lesquels R est un radical alkyle linéaire ou ramifié en C₁₀ à C₁₄.

3. Sels selon la revendication 2, dans lesquels R est un radical alkyle linéaire en C₁₃.

4. Utilisation de sels selon l'une quelconque des revendications 1 à 3, comme agents bactériostatiques, mycostatiques, statiques vis-à-vis de la levure et/ou microbicides.

5. Formulations comprenant au moins un sel selon l'une quelconque des revendications 1 à 3, dans lesquelles ledit au moins un sel est à des concentrations comprises entre 0,001 et 1 % en poids par rapport au poids de la formulation totale.

6. Formulations selon la revendication 5, dans lesquelles ladite concentration est comprise entre 0,05 et 0,1 %.

7. Formulations selon la revendication 5 ou la revendication 6, destinées au nettoyage ou à la purification de la peau ou d'une muqueuse, lesdites formulations étant à base d'eau.

8. Formulations selon l'une quelconque des revendications 5 à 7, lesdites formulations se présentant sous forme de savons anti-acné, de lotions ou d'émulsions.

9. Formulations selon l'une quelconque des revendications 5 à 7, lesdites formulations se présentant sous forme de shampooing anti-pelliculaire.

10. Procédé de préparation de sels de formule générale (I) comprenant les étapes suivantes consistant à :
a) estérifier du diméthylaminoéthanol avec un halogénure de formule RCOX', où X' est un atome d'halogène choisi parmi le chlorure ou le bromure, obtenant ainsi l'ester de formule (II), 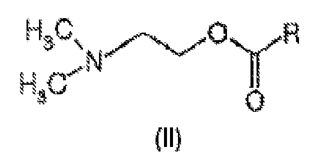 dans laquelle R est un radical alkyle linéaire ou ramifié en C₆ à C₁₆,
b) salifier l'ester de formule (II) par traitement du mélange de réaction dérivé de l'étape (a) avec l'acide HX", où X" = I, afin d'obtenir les sels de formule (I).

11. Procédé selon la revendication 10, dans lequel l'étape (a) est réalisée en présence d'un accepteur d'ions hydrogène.

12. Procédé selon la revendication 11, pour préparer le sel de formule (I) dans lequel X⁻ = I⁻, dans lequel les étapes (a) et (b) sont réalisées dans un solvant aqueux et dans l'étape (a), de l'hydroxyde de sodium est utilisé en tant qu'accepteur d'ions hydrogène, et à l'achèvement de l'étape (b) après addition d'une quantité stoechiométrique d'acide iodhydrique, le sel précipite directement du mélange de réaction aqueux lors du refroidissement.
